# EUROPEAN PATENT SPECIFICATION

(11) **EP 4 536 133 B1**
(45) Date of publication and mention of the grant of the patent: **05.11.2025**
(21) Application number: 23728782.6
(22) Date of filing: 29.05.2023
(51) Int. Cl.: A61C 17/00, A61C 19/04, A61C 9/00, A61C 17/22

(54) **DETERMINING A MOUTH OPENING VALUE**
BESTIMMUNG DES WERTES FÜR DIE MUNDÖFFNUNG
DÉTERMINATION D'UNE VALEUR D'OUVERTURE DE LA BOUCHE

(30) Priority: 10.06.2022 US 202263350881 P; 19.07.2022 EP 22185668
(43) Date of publication of application: 16.04.2025
(73) Proprietor: Koninklijke Philips N.V., 5656 AG Eindhoven (NL)
(72) Inventor: NUNES, Dionisio Massingo, 5656 AG Eindhoven (NL); LIPTAK, Richard William, 5656 AG Eindhoven (NL); JOHNSON, Mark Thomas, 5656 AG Eindhoven (NL); GERHARDT, Lutz Christian, 5656 AG Eindhoven (NL)
(74) Representative: Philips Intellectual Property & Standards
(86) International application number: PCT/EP2023/064309
(87) International publication number: WO 2023/237369

(56) References cited:
- EP-A1- 3 957 275
- EP-A1- 4 070 759
- WO-A1-2018/037318
- WO-A1-2019/215318
- US-A1- 2007 270 221
- US-A1- 2011 010 875
- US-A1- 2019 200 746
- US-A1- 2020 359 777

## Description

### FIELD OF THE INVENTION

The invention relates to the field of oral health. In particular, the invention relates to the field of detecting trismus and temporomandibular joint dysfunction.

### BACKGROUND OF THE INVENTION

Trismus (commonly known as Lockjaw) and temporomandibular joint (TMJ) dysfunction (TMJD) cause the reduced opening of the jaws or the limited motion thereof. Trismus and TMJD can interfere with eating, speaking, and maintaining proper oral hygiene. When left untreated or undiagnosed, trismus and TMJD can become chronic and painful, as well as affecting aspects of a person's daily life and health by causing migraine headaches, discomfort in the back, neck, and shoulder and hearing damage/loss.

Examination and treatments of trismus and TMJD typically require access to the oral cavity which can be limited or, in some cases, unfeasible. Trismus can be caused by joint issues, infections, trauma, cancer therapies, or post-dental treatments. For example, temporomandibular joint diseases can lead to trismus. Around 12% of the population in the United States (around 35 million people) are affected by TMJD at any given time.

Another notable cause of trismus is head and neck cancer, where the prevalence of trismus is estimated to be up to 38% in these cases. For patients suffering from this type of cancer, trismus management focuses on preventing the progression of trismus and restoring mandibular function. Currently, there are no non-invasive mechanisms to track the progression of trismus or track the restoration of mandibular function to allow health professionals to see how their patients respond to treatments.

A common approach to diagnosing the severity of trismus is to measure a subject's mouth opening via a tool, such as a Boley gauge or a ruler. A normal oral opening (i.e. no trismus) is expected to be above 40 mm. Mild trismus is typically categorized based on a mouth opening of between 30 - 40 mm, moderate trismus is typically categorized based on a mouth opening of between 15 - 30 mm and severe trismus is typically categorized based on a mouth opening of below 15 mm.

Alternatively, a three finger screening test can be used to approximate the severity of trismus. In the three finger screening test, a subject attempts to open their mouth as much as possible to accommodate their fingers between the top and bottom teeth. Being able to accommodate three or more fingers typically indicates no trismus, two to three fingers indicates mild trismus, one to two fingers indicates moderate trismus and less than one finger indicates severe trismus.

Both approaches are considered invasive, require a second person to do the measurement, and do not provide health professionals the tools needed to monitor the patient's progress after a procedure, such as a wisdom tooth extraction.

WO2019215318 discloses a personal care device using sensors and a controller with feature extraction and classification capabilities to assess a user's status and adjust its functions accordingly for tailored care.

US5097820A discloses the construction of a device that can be used to diagnose and treat a limited mouth opening. However, such devices tend to be invasive and expensive. WO2017015342A1 discloses a trismus rehabilitation device similar to the device disclosed in US5097820A with additional sensors to measure the pressure applied at various points of the jaw to estimate the mouth opening.

A major limitation of these solutions is that their purpose is limited to single-spot measurements usually done by a dental professional in the practice or clinic, resulting in infrequent use of these solutions by the subject. This reduces their helpfulness in large scale monitoring of TMJD or trismus. Additionally, it is difficult to digitize these measurement in a seamless workflow to enable long-term monitoring of disease progression.

Thus, there is a need for improved means to detect the presence of trismus or any mouth opening/closure disorder and continuously track its progress, particularly in a home environment.

### SUMMARY OF THE INVENTION

The invention is defined by the claims.

According to examples in accordance with an aspect of the invention, there is provided an oral care system comprising:
an oral care device comprising a sensor system configured to measure one or more parameters of the oral care device or of the user's mouth during use of the oral care device; and
a processor configured to obtain the one or more parameters from the sensor system and determine a mouth opening value of the user based on the one or more parameters.

The oral care device is not for treating mouth opening disorders (e.g. trismus). For example, the oral care device may be for maintaining oral hygiene or a treatment device for treating a second oral health disorder which is not a mouth opening disorder.

Trismus, typically known as lockjaw, is a condition which limits the motion of the jaw and can interfere with eating, speaking or maintaining oral hygiene. Current approaches for detecting trismus and determining the severity of trismus comprise measuring the user's mouth opening using a Boley gauge, a ruler or, in some cases, using the number of fingers which fit in the mouth opening. However, the finger measurements are not particularly accurate and have a very limited resolution whilst the more precise measurements are difficult to perform by oneself.

Thus, it has been realized that trismus, or any other cause of limited mouth openings, can be checked when the user is maintaining oral hygiene. For example, brushing teeth or using a jetting device is typically performed daily. This increases the amount of data obtained which is relevant to the detection of trismus without the user having to add additional activities to their daily routine or having to perform difficult or uncomfortable tests.

The idea is that, when using most oral care devices, the user is bound to open their mouths as much as they can at some point. Thus, a sensor system can be added to the oral care device which is configured to obtain parameters indicative of the oral care device or of the user's mouth, thereby providing an indication of how much the user's mouth can open. The measured parameters may be suitable for determining how much the user is opening their mouth during use of the oral care device.

The mouth opening value is a value (or set of values) indicative of how much a user is physically opening their mouth. The method may comprise determining the maximal mouth opening value.

The maximal mouth opening value is a value (or set of values) indicative of how much a user can physically open their mouth. Typically, the maximal mouth opening value is the measurement of the distance between top and bottom front teeth. However, any distance at any point in the mouth is also indicative of the mouth opening. Similarly, it has been realized that the maximum angle the top and lower jaws form when the mouth is opened is also indicative of how much a user can physically open their mouths. Thus, the maximal mouth opening value may be a distance measurement relative to a mouth position, or an angle measurement.

A minimal mouth opening could also, or alternatively, be determined. The minimal mouth opening is a value (or set of values) indicative of how much a user can physically close their mouth. This information could be helpful in the eventual identification of mouth closure disorders. The minimal mouth opening could be referred to as a maximal mouth closure.

The mouth opening value may be determined when the user is performing a particular action. The method may comprise determining whether the user is performing a particular action and use the parameters corresponding to when the user is performing the action. The particular action may vary depending on the oral care device chosen and the desired mouth opening value. For example, when using a jetting device, the mouth may be closed when the user is jetting the top front teeth which may allow the determination of the minimal mouth opening. The particular actions may include brushing and/or jetting a particular area of the mouth.

The oral care device may be an oral care device for maintaining oral hygiene. Alternatively, the oral care device may a treatment device.

At least one of the one or more parameters may be the orientation of the oral care device in the mouth of a user and wherein the processor is further configured to detect changes in orientation of the oral care device using the orientation measurements from the sensor system and determine the mouth opening value of the user based on the changes in orientation.

Detecting the changes orientation provides a proxy measurement for the angle at which the mouth is opened. It has been found that the maximal angle by which a user can open their mouth provides a strong indication for potential trismus, or other conditions which limit mouth openings. Thus, the maximal mouth opening may be measured in a maximal angle instead of the typical distance measurements between the incisors.

The orientation may comprise at least one angle measurement. The angle measurements could be relative to gravity and/or relative to the tooth. For example, the angle measurements may comprise a pitch angle measurement, a roll angle measurement and/or a yaw angle measurement of the oral care device. In some cases, changes in the pitch angle measurements may be sufficient to determine the mouth opening value.

At least one of the one or more parameters may be the motion of the oral care device in the mouth of a user and wherein the processor is further configured to determine the mouth opening value of the user based on the motion of the oral care device.

The motion of the oral care device can also provide suitable measurements for determining the maximal mouth opening. For example, the motion of the oral care device from the top teeth to the bottom teeth may provide an indication of the maximal mouth opening.

The processor may be further configured to determine the location of the oral care device in the mouth of the user.

At least one of the one or more parameters may be the location of the oral care device in the mouth of the user. Thus, the processor could determine the location of the oral care device by obtaining the location of the oral care device in the mouth of the user from the sensor system.

Alternatively, the processor could process the one or more parameters to determine the location of the oral care device in the mouth of the user. For example, a distance sensor could be used to measure the maximal distance to the back of the mouth and determine the location by comparing the current distance to the maximal distance to the back of the mouth.

The processor may be further configured to determine whether the oral care device is being used on the rearmost teeth of the upper jaw of the user, and wherein determining the mouth opening value is based on the measurements from the sensor system which correspond to the oral care device being used on the rearmost teeth of the upper jaw of the user.

It has been found that when the user is using an oral care device on the rearmost teeth (e.g. wisdom tooth) of the upper jaw, this naturally causes the jaw to drop to a maximum, thereby causing their mouth to open to a maximum. Thus, measurements taken at this point are very likely to be indicative of the maximal mouth opening.

The processor, using the one or more parameters from the sensor system, may be configured to determine whether the oral care device being used on the rearmost teeth of the upper jaw of the user by determining the oral care device is positioned on the upper jaw, determining the oral care device has moved towards the back of the mouth and determining the oral care device is positioned on the occlusal side of the rearmost teeth of the upper jaw.

At least one of the one or more parameters may be a depth of the oral care device within the mouth of the user and wherein determining the mouth opening value is further based on the depth of the oral care device within the mouth of the user.

The depth of the oral care device provides context of where the oral care device is within the mouth of the user. In the case where the sensor system provides motion measurements of the oral care device, the depth within the mouth can be used to translate the measured motion at the measured depth to the corresponding motion at a depth of zero (i.e. at the front of the mouth where the maximal mouth opening is typically measured.

Additionally, any measurements from the sensor system could be compared to the previous measurements corresponding to the same depth in order to track potential conditions limiting the maximal mouth opening.

The processor may be further configured to account for the size of the oral care device when determining the mouth opening value.

The size of the oral care device may include the thickness, the diameter, the width, the height and/or the length of the oral care device.

The processor may be further configured to track the one or more parameters from the sensor system over time for the user and track a progress of the user's mouth opening based on the changes in the one or more parameters over time.

The processor may be further configured to track the one or more parameters from sensors systems in a plurality of oral care devices used by the user, wherein tracking the progress of the user's mouth opening comprises combining the tracked one or more parameters for the plurality of oral care devices, wherein, during the combination, the one or more parameters are weighted based on the corresponding oral care device.

The processor may be further configured to determine how much time has passed since the latest determined mouth opening value and alert the user to use the oral care device if the time is larger than a threshold time period.

The oral care device may be a toothbrush, a jetting device or a toothbrush with a jetting device.

The sensor system may comprise one or more of an accelerometer, a rotation sensor, a displacement sensor, a location sensor, a force sensor, a pressure sensor, a torque sensor and an angulation sensor, a distance sensor.

The sensor system may comprise an acoustic sensor for measuring sounds in the mouth of the user, wherein determining the mouth opening value is based on the sounds in the mouth of the user.

At different mouth opening values, the oral cavity of the user forms a chamber of different volumes. Sound resonates differently in different volume shapes and sizes. Thus, the characteristics of sound resonating the oral cavity are dependent on the mouth opening of the user.

When the mouth is opened wider (or closed), the sound spectrum of the brush is expected to change. However, when the head of the user is tilted and the mouth stays closed (or open), the sound spectrum would remain unchanged. Thus, the acoustic sensor may also be used in determining whether a change in parameters corresponds to the head of the user being tilted or the opening of the mouth.

When using a powered oral care device, the drive train dampening of the oral care device when it is being used may also be acoustically measured to inform whether the mouth is open.

The sensor system may comprise a distance sensor and/or a proximity sensor placed at a distal portion of the oral care device, wherein the distal portion of the oral care device is the portion of the oral care device inserted into the mouth of the user.

For example, the distal portion may be the brush head of a toothbrush.

The invention also provides a method for determining a mouth opening or closing of a user, the method comprising:
obtaining one or more parameters, from a sensor system on an oral care device, the parameters being of the oral care device or of the user's mouth during use of the oral care device; and
determining a mouth opening value of the user based on the one or more parameter.

The invention also provides a computer program product comprising computer program code which, when executed on a computing device having a processing system, cause the processing system to perform all of the steps of the afore-mentioned method.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Figure 1 illustrates an open mouth;
Figure 2 shows a toothbrush head with a sensor system at the head of the toothbrush;
Figure 3 shows a toothbrush head with a sensor system being used on a mouth;
Figure 4 shows a mouth with isosceles triangle;
Figures 5 and 6 show a toothbrush being used on the rearmost teeth of the upper jaw;
Figure 7 shows a toothbrush being used on the lingual side of the top front teeth;
Figure 8 shows an image used to determine a mouth opening; and
Figure 9 shows a method for determining an absolute mouth opening

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides an oral care system comprising an oral care device and a processor. The oral care device has a sensor system which is configured to measure one or more parameters of the oral care device or of the user's mouth whilst the oral care device is being used on the user. The processor is configured to obtain the one or more parameters from the sensor system and determine a mouth opening value of the user using the one or more parameters.

A system is proposed to obtain a mouth opening of a user by using an oral-care device. This, for example, enables a clinician to detect when a user has mouth opening dysfunctions and, through continued use of the system, estimate its progression.

This facilitates the continuous remote monitoring of TMJ dysfunction or trismus, allowing health and dental professionals to assess and quantify the outcome of the treatments they provide. An additional benefit is that health and dental insurers will have additional data points to verify benefits claim related to TMJ disorder or trismus, by assessing claimed treatment dates with the dates of in which trismus was detected.

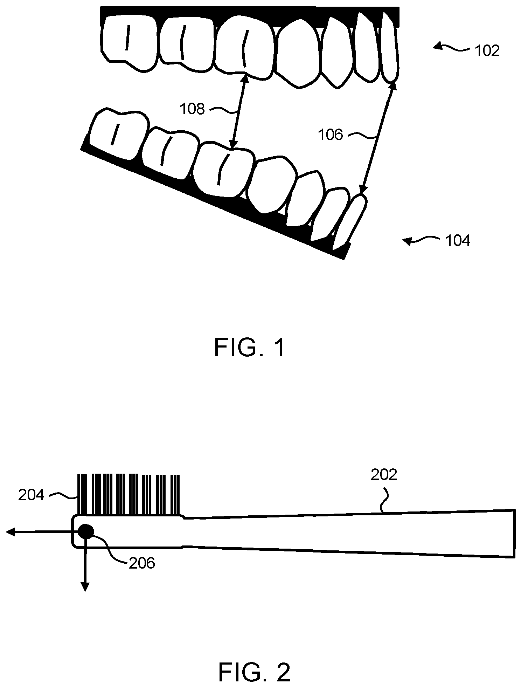

Figure 1 illustrates an open mouth. As can be seen, the upper teeth 102 do not separate from the bottom teeth 104 uniformly and thus the mouth opening (lines 106 and 108) can differ depending on where the mouth opening measurements are taken. Currently, the mouth opening values/measurements are mostly taken at the front of the mouth (i.e. line 106) as this is the most readily available measurements from the outside and thus a clinician, or other person, can measure mouth opening of the user.

However, it will be appreciated that the mouth opening values can be obtained at any location within the mouth in a manner which can be interpreted by a clinician. For example, line 108 (near the middle of the mouth) may be used in combination with the particular location within the mouth. In contrast, when the location is not known, a processor may obtain many measurements and find the maximum value of those measurements. The maximum measurement will typically be interpreted as the mouth opening at line 106 (at the front of the mouth) as this is typically the location with the largest mouth opening.

A maximal mouth opening is currently one of the most relevant mouth openings measured. The maximal mouth opening quantifies by how much a user can open their mouth. A relatively small maximal mouth opening could indicate the possibility a mouth opening disorder. Similarly, a minimal mouth opening (i.e. a maximal mouth closure) is also a relevant mouth opening. If a user cannot fully close their mouth, this would result in a relatively large minimal mouth opening, thereby indicating the possibility of a mouth opening disorder.

It is noted that Figure 1 shows a generic mouth opening. In practice, a maximal mouth opening (and potentially a minimal mouth opening) are typically of more clinical relevance than a generic mouth opening. However, a whole range of mouth openings may be of relevance if accurate and frequent mouth openings are obtained.

The inventors have realized that using oral care/hygiene devices (e.g. toothbrush, flossing device, jetting device etc.) are all used in the user's mouth and are typically used frequently (e.g. one or more times a day). Additionally, given the nature of using the oral care devices in the mouth of the user, the user will typically open their mouth during use. Thus, it is proposed to measure the mouth opening of a user whilst using an oral care device. This enables the user to do two things can be done at once (i.e. maintain oral hygiene and measure mouth opening). It also means that mouth opening values are likely to be obtained much more frequently than if the user or, typically, a clinician had to separately perform the mouth opening measurement.

The following examples will be in relation to measuring the maximal mouth opening. However, it will be appreciated that systems and methods described in relation to measuring the maximal mouth opening can also apply to the measurement of any generic mouth opening. In specific cases where the systems or methods relate specifically to the maximal mouth opening, alternatives may be given for the minimal mouth opening or generic mouth openings.

Similarly, the following examples will use a toothbrush to illustrate the examples. However, it will be appreciated that other oral care devices may also be used.

In a first embodiment, sensors are placed at the head of a toothbrush. Figure 2 shows a toothbrush head 202 with a sensor system 206 at the head of the toothbrush 202. The sensor system is placed at a distal end of the toothbrush head 202 near the bristles 204 of the toothbrush head 202. In an alternative embodiment, the sensor system can be also present in the handle of an oral care device.

Quantification metrics for mouth opening may depend on where along the patient's jaw the measurements are taken. This is because the mouth opens widest at the anterior and narrowest at the posterior.

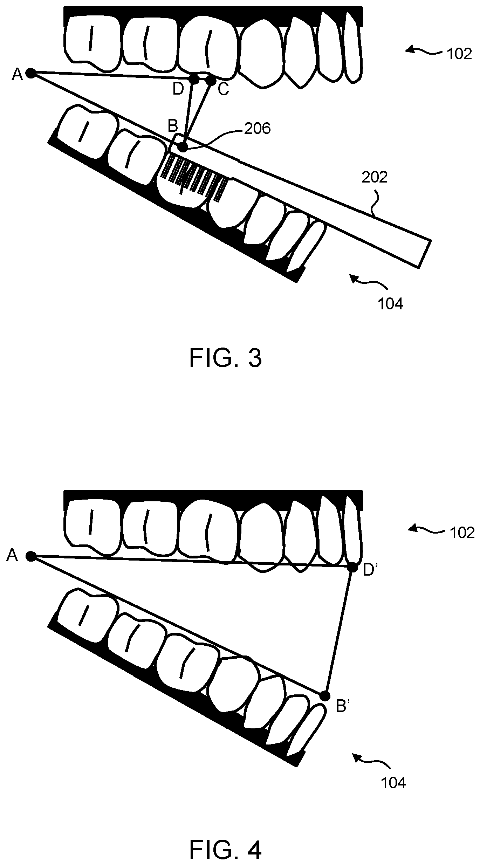

Figure 3 shows a toothbrush head 202 with a sensor system 206 being used on a mouth. In this case, the sensor system may comprise proximity sensors and/or distance sensors and/or acceleration sensors (inertia measurement) and/or a gyroscope. In order to estimate the level of mouth opening at any location in the mouth, the sensor system determines the position of the brush head along a given jaw from distance measurements taken by the sensor system 206 attached on its longitudinal axis (i.e. along segment AB shown in Figure 3). The mouth opening at the current brush-head position is determined from distance measurements taken from the sensors attached on the vertical axis (i.e. along segment BC shown in Figure 3).

An angle of elevation can be computed at the current position (i.e. the angle ∠BAC = ∠BAD). As can be seen, the isosceles triangle BAD can also be used. Point D can be found using simple geometry from point A, B and C.

Those skilled in the art will appreciate that calculation of segment BD is straightforward. Given measurement of segment BC, an approximate isosceles triangle (ABD) can be created from which basic trigonometric functions allows the deduction of BD. For this, segment AD is calculated by a simple application of Pythagoras's theorem on triangle ABC.

While the planes of both the upper and lower jaws are not linear, by taking measurements when the brush head is as far into the posterior of the occlusal plane as possible, local linearity can be reasonably expected such that the mouth opening that far in the mouth can be approximated by calculating the distance represented by line segment BD.

The mouth opening at any other points, including maximal opening, can therefore be estimated using the angle of elevation. Estimating the opening of a point elsewhere is possible given knowledge of both BD and the angle ∠BAD at that particular given point. It is expected that the nonlinear plane both jaws may limit the precision of the proposed approach. However, the range of mouth opening is great enough (e.g. 15 mm for moderate trismus) that an estimate made using this method is expected to be well within acceptable range.

For example, Figure 4 shows a mouth with isosceles triangle (AB'D'). As can be seen, the triangle surprisingly fits the shape of the open mouth despite the irregular nature of teeth shapes. As such, one can conclude that the isosceles triangle is a good descriptor of a mouth opening. This leads to the conclusion that the angle ∠B'AD' = ∠BAD can also be accurately used to represent the mouth opening. As such, the mouth opening value is not limited to a distance measurement between the upper teeth 102 and the lower teeth 104 and the angle of elevation can also be used as the mouth opening value.

The first embodiment may be realized with a sensor system comprising a two-sensor configuration in which the sensitive axis of both sensors are perpendicular to each other. The direction of the sensors is indicated by segments BA and BC in Figure 3. In general, the first example uses triangulation to estimate the mouth opening value.

In a second embodiment, a mouth opening value is determined using sensor data from a sensor system already present in current toothbrushes. In this embodiment, the measurements are taken at unique brushing locations and orientations (e.g. behind the upper or lower molars). In particular, the second embodiment enables measurements to be taken during precise and unique actions which result in a desired mouth opening (e.g. maximal or minimal mouth opening).

Whilst the first embodiment makes use of sensors added to the brush head in order to facilitate a triangulation measurement of mouth opening, it has been realized that it is also possible to evaluate and track the opening of the mouth using sensors already present on a (powered) oral health care device. For example, accelerometers, inertial measurement units, rotation sensors (e.g. compass, gyroscope etc.), displacement sensors, location sensor, hall sensor and/or printed strain gauge may already be present in a powered oral health care device.

As a reminder, the description and figures show a powered toothbrush used as an example. However, any other toothbrush (e.g. combined brushing flossing device) or water jetting device or flossing device could be considered. In particular a water jetting device is extremely suitable as the head of the device with jetting tube is rather long and narrow which can reduce measurement issues (e.g. no issue with a wider device body of a toothbrush getting in the way of the measurements).

It has been realized that during a specific brushing motion - namely the brushing behind the rearmost molars/wisdom teeth - the user is obliged to open their mouth to the maximum extent and to rotate the brush through an angle until it is pressed hard against the opposite jaw to the tooth being brushed.

It is the object of this embodiment to measure this brush motion and to interpret this measurement as representative of the opening of the mouth. Limited motion, or a reduction of opening with time can relate to mouth opening disorders (e.g. TMJ, trismus etc.).

Whilst measurements taken during cleaning of the top or bottom teeth can in principle both lead to an interpretation of the mouth opening, the inventors have realized that it may be preferable to use measurements whilst brushing behind the rearmost molars/wisdom teeth in the upper jaw.

Whilst brushing the upper jaw, the opening of the mouth causes the jaw to drop, leaving the brush initially in the same position flat against the upper teeth as it was whilst brushing along the top of the upper teeth. From this fixed reference position, the brush must traverse the full opening angle of the mouth before it presses against the lower jaw. As a consequence the measured motion is a direct measure of the opening of the mouth.

In contrast, whilst brushing the lower jaw, the opening of the mouth again causes the jaw to drop. However, in this case the jaw drop already separates the brush from the jaw without any requirement to move the brush. From this starting position, the brush is only required to traverse a fraction of the opening angle of the mouth before it presses against the upper jaw. As a consequence the measured motion may not be a direct measure of the opening of the mouth. However, it must be noted that this measurement may have some predictive value for longitudinal measurements.

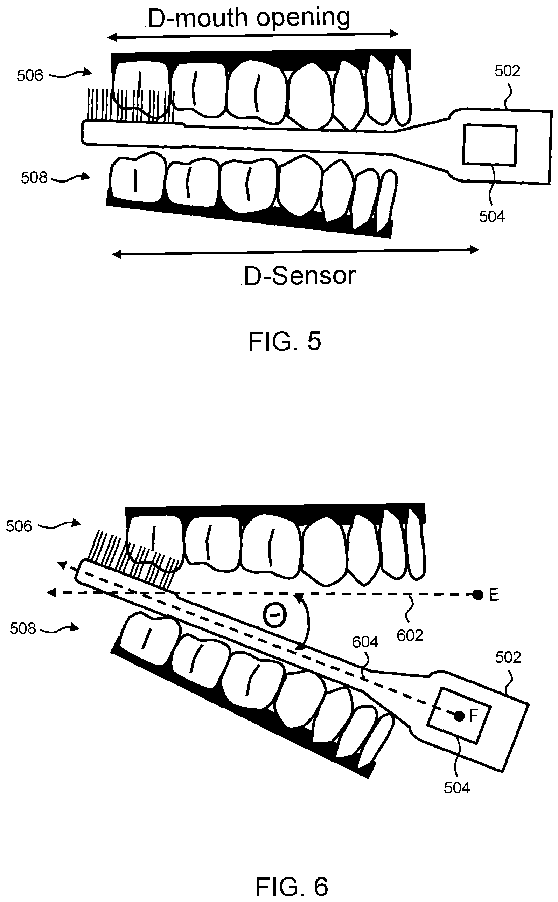

Figures 5 and 6 show a toothbrush 502 being used on the rearmost teeth of the upper jaw 506. In order to establish the opening of the mouth during brushing of the upper jaw 506 the following measurements are suitable measures:
- Measurement of the total movement of the sensor system 504 (e.g. including an accelerometer and/or a position sensor) as the brush rotates from upper jaw 506 (as shown in Figure 5) to the lower jaw 508 (as shown in Figure 6).
- Measurement of the angle of rotation of the sensor system 504 (e.g. including a compass and/or a gyroscope) as the toothbrush 502 rotates from upper jaw 506 (as shown in Figure 5) to lower jaw 508 (as shown in Figure 6). If desired, the rotation angle can be converted into a movement amplitude by considering the distance of the sensor system 504 from the top of the brush head to the sensor system 504.

For many use cases (e.g. such as establishing the progression of TMJD) it is sufficient to monitor changes of the measured brush sensor motion or angle of rotation.

However for some use cases (e.g. such as establishing the severity of the TMJD/trismus) it may be preferred to establish the absolute opening of the mouth. In order to achieve this, the magnitude of the distance traversed by the brush at the position on the brush where the brush leaves the mouth may be established. This can be derived as follows:
The brush motion at mouth opening = measured motion of sensor system 504 between point E and F in Figure 6 × the ratio between the depth of the mouth (D-mouth opening) and the distance between the sensor system 504 (D-Sensor) and the brush head. The depth of the mouth (D-mouth opening) is the distance between the foremost teeth of the mouth and the rearmost teeth of the mouth.

The size (e.g. diameter, thickness etc.) of the brush at the position on the brush where the brush leaves the mouth (diameter brush) may be taken into consideration when calculating the absolute mouth opening: For example, mouth opening = Distance traversed by the brush at the position of the mouth opening + brush diameter.

Note that for this absolute mouth opening measurement it is advantageous if the portion of the toothbrush entering the mouth is relatively narrow with a constant diameter. For example, a relatively longer toothbrush which protrudes from the mouth even when placed behind the last tooth. A jetting device may be particularly suitable as it has an inherently long and thin tube for jetting purposes.

It is also possible to establish the most likely moment to associate a brush motion to a mouth opening. Specifically, if the sensor system 504 comprises a motion or location sensor (or similar) the following pattern is likely to represent a preferred measurement moment:
- Brush is positioned to brush the top surface of the upper teeth;
- The brush moves backwards along the teeth;
- Either the brush stops moving (motion sensor) or the location is at or behind the rear molar/wisdom tooth (location sensor) and remains constant; and
- A motion of the brush is measured representing a possible mouth opening due to jaw drop;

In a particular use case, it may be establish that a specific brush head is being used specifically to brush behind the rearmost tooth (i.e. a single tuft brush head). A higher weighting may be given to measurements made for this particular use case as it is most likely users are focusing on cleaning behind the molar and will try to fully open their mouth

Clearly, it is not necessary that the user carries out the maneuver every time they are brushing, as these jaw related problems only develop slowly in time. However if a relatively long period has occurred since a measurement was possible, in an alternative approach, the user may be instructed to carry out this specific motion by placing the tip of the device as described above, opening their jaw and moving the device parallel to the opening of the jaw. In such a manner a good quality reference measurement can always be established. The user may be instructed via an external device (e.g. an app on their smartphone).

Existing oral care devices use an algorithm to determine the orientation of the oral device with respect to the gravity vector. This could be used to determine the change in angle when opening the mouth. The orientations 602 and 604 are shown in Figure 6. The orientation 602 is the orientation of the toothbrush as illustrated in Figure 5. Point E shows the placement of the sensor system 504 corresponding to the orientation 602 of the toothbrush. Similarly, orientation 604 is the orientation of the toothbrush as illustrated in Figure 6 corresponding to the sensor system 504 being at point F. Thus, the angle Θ between the two orientations can be found.

In some cases, the conversion from the angle measurement to the distance of the mouth opening (requiring 'jaw length') may not be required here. In fact, the angle of the mouth opening may be more accurate (as it requires less measurements) and possibly more informative compared to the distance measurement of the mouth opening. It may be that the distance measurements (typically in mm) of the mouth opening may be commonly used as it is easiest to assess with current practices.

Robustness to head motion may also be a point of attention. When detecting an orientation change, a mouth opening could be confused with head tilting. One approach to address this could be to use a trained an algorithm to distinguish between 'mouth opening' and 'head tilting'. Algorithms such as these are currently used for determining the location of a toothbrush in the mouth.

For example, location sensing algorithms using inertia measurement unit (IMU) sensor signals have been previously used. These algorithms use an average, or median, direction of the brush over time to estimate the head tilt. The head tilt is then used to get a better estimate of the brush orientation with respect to the head of the user. In some cases, the direction of the head tilt can be re-estimated (based on the brush direction) each time the user changes to actions to another segment of the mouth.

A second approach could be to use a pressure sensor to determine when the brush head is pushed against the teeth and when the mouth opening is due to 'brushing behind the molars'. A third approach could be to add an acoustic sensor to the sensor system. Increased sound level and spectral changes occur due to mouth cavity resonances. These will differ based on whether the mouth is open or closed.

In a third embodiment, an image algorithm with dynamic image re-scaling and calibration is used to determine a (maximum) mouth opening at a unique brushing location and orientation (lingual incisor brushing).

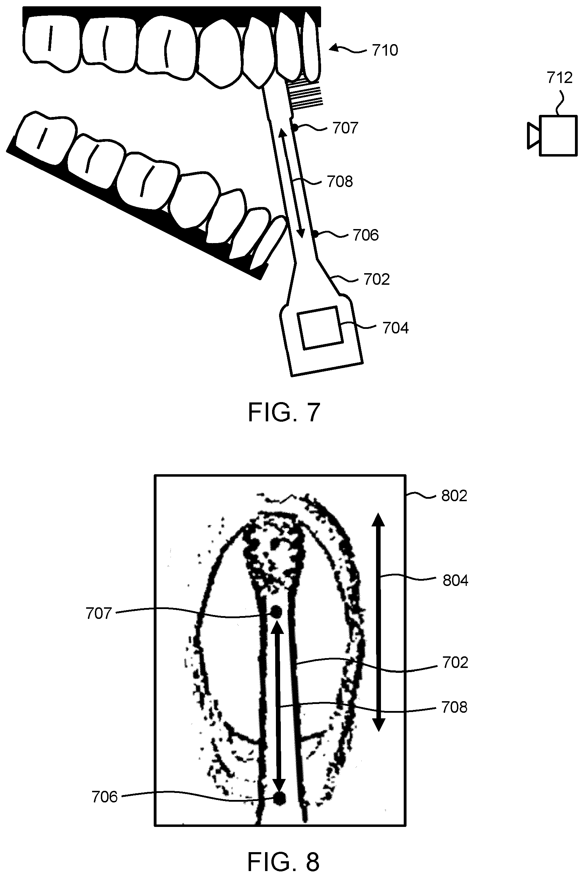

Figure 7 shows a toothbrush 702 being used on the lingual side of the top front teeth 710. The toothbrush comprises two markers 706 and 707 with a known length 708 between the markers 706. A camera 712 is used to take images of this action.

The third embodiment provides software-enabled measurements of mouth opening by using calibrated toothbrush (or portion thereof) and a camera (e.g. on a smart phone) without the requirement for sensors on the toothbrush 702. The camera could be also part of the toothbrush handle (distal end) while, during brushing, the camera can face the bathroom mirror and in such a way image the mouth opening.

The mouth opening is measured at the shown unique brushing orientation/location which provides a reliable mouth opening width measurement. It has been realized that during specific brushing motions (e.g. brushing behind the maxillar or mandibular incisors), the user is obliged to open their mouth to the maximum extent and to rotate the brush through a unique angle until it is pressed hard against the lingual upper or lower jaw to the tooth being brushed as shown in Figure 7.

By knowing the distance between the (at least) one (reference) marker 706 of a length calibrated toothbrush shaft and identifying the markers and mouth opening (e.g. using a feature detection algorithm and/or feature matching algorithm), one can determine the absolute mouth opening by for example accessing a database with known brush head or reference marker dimensions to derive image calibration or image scaling factors. The marker(s) 706 could be a colored/textured dot added to the toothbrush or markers inherent in brush head (e.g. ejector marks from injection molding process). A single marker 706 (e.g. of known diameter) could be used.

A database containing the reference length 708 can be accessed, thereby allowing one to dynamically rescale the image. The known length enables the determination of the absolute maximal mouth opening values reliably (e.g. if images are taken at subsequent weeks), as one cannot reliably control the distance from which image is taken (i.e., a compensation and auto-scaling for different image magnification factor is performed).

The toothbrush 702 may be connected with database of brush head design data (e.g. drawings and dimensions) including the dimensions (e.g. in mm) of the toothbrush (or a portion thereof). A radio frequency Identification (RFID) code could be used to recognize the type of toothbrush attached.

An image is acquired whilst the toothbrush is being used to allow one to determine the mouth opening. Figure 8 shows an image used to determine a mouth opening. The user may brush their teeth and takes images (e.g. via a smartphone camera) when the incisors are brushed. Alternatively, a camera could be part of a bathroom mirror.

A processor, or cloud-engine, then executes the following algorithm steps to determine an absolute maximal mouth opening from the acquired image as shown in a processed image in Fig. 8:
- Decompose R-G-B image into R-G-B channels;
- Apply a feature recognition function (e.g. boundary and edge detection) to differentiate between brush head features and oral cavity features (e.g. lips, teeth) - see Fig. 8;
- Identify markers 706 and 707 and measure relative distance (measured in pixels) between both markers;
- Identify a relative mouth opening 804 (measured in pixels);
- Re-scale the uploaded image based on boundary segmented features and known distance 708 (e.g. measured in mm) of markers 706 on brush head and the relative measurements;
- A calibration factor can be determined (e.g. distance/pixel) using the relative distance of the identified markers 706 and 707 in the image and the known distance 708; and
- Apply the length calibration factor to the relative mouth opening 804 to determine absolute maximum mouth opening.

Optionally, a sensor system 704 already present in the toothbrush 702 can be used to improve the accuracy of the measurements by taking into account brushing angle influences on the determination of the absolute mouth opening. It is noted that the mandibular incisors and maxillary incisors are brushed at different pitch angles.

The camera 712 could also be placed on the toothbrush 702. This takes advantage of the fact that users typically brush their teeth in front of a mirror. Thus, a camera 712 can be placed on the toothbrush such that it takes an image of a mirror in front of the user reflecting the user brushing their teeth. This would also negate the need to use an external camera when brushing teeth.

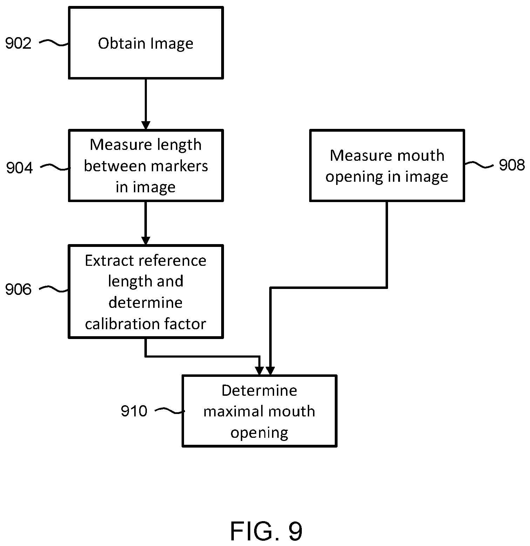

Figure 9 shows a method for determining an absolute mouth opening. The method comprises receiving images of the user brushing their teeth in step 902, measuring the (relative) length (in pixels) between markers (or other portions) on the toothbrush in step 904 and determining a calibration factor 906 (e.g. in pixels/mm or distance/pixels) using the measured length between the markers and a known length between the markers in step 906, e.g. extracted from a database. The (relative) mouth opening is also determined or measured (in pixels) from the image in step 908. Thus, the calibration factor can be applied to the (relative) mouth opening to determine an absolute mouth opening. Note that the mouth opening may be a maximal mouth opening, a minimal mouth opening or any generic mouth opening.

The skilled person would be readily capable of developing a processor for carrying out any herein described method. Thus, each step of a flow chart may represent a different action performed by a processor, and may be performed by a respective module of the processor.

As discussed above, the system makes use of processor to perform the data processing. The processor can be implemented in numerous ways, with software and/or hardware, to perform the various functions required. The processor typically employs one or more microprocessors that may be programmed using software (e.g., microcode) to perform the required functions. The processor may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs).

In various implementations, the processor may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

Functions implemented by a processor may be implemented by a single processor or by multiple separate processing units which may together be considered to constitute a "processor". Such processing units may in some cases be remote from each other and communicate with each other in a wired or wireless manner.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa.

Any reference signs in the claims should not be construed as limiting the scope.

## Claims

1. An oral care system comprising:
an oral care device (202, 502) comprising a sensor system (206, 504) configured to measure one or more parameters of the oral care device or of the user's mouth during use of the oral care device; and
a processor configured to obtain the one or more parameters from the sensor system and determine a mouth opening value (106, 108) of the user based on the one or more parameters; and
wherein at least one of the one or more parameters is the orientation (602, 604) of the oral care device in the mouth of a user and wherein the processor is further configured to:
detect changes in orientation of the oral care device using the orientation measurements from the sensor system; and
determine the mouth opening value of the user based on the changes in orientation; and/or
wherein at least one of the one or more parameters is the motion of the oral care device in the mouth of a user and wherein the processor is further configured to determine the mouth opening value of the user based on the motion of the oral care device.

2. The system of any one of claims 1, wherein the processor is further configured to determine the location of the oral care device in the mouth of the user.

3. The system of any one of claims 1 to 2, wherein the processor is further configured to determine whether the oral care device is being used on the rearmost teeth of the upper jaw (506) of the user, and wherein determining the mouth opening value is based on the measurements from the sensor system which correspond to the oral care device being used on the rearmost teeth of the upper jaw of the user.

4. The system of claim 3, wherein the processor, using the one or more parameters from the sensor system, is configured to determine whether the oral care device being used on the rearmost teeth of the upper jaw of the user by:
determining the oral care device is positioned on the upper jaw (102);
determining the oral care device has moved towards the back of the mouth; and
determining the oral care device is positioned on the occlusal side of the rearmost teeth of the upper jaw.

5. The system of any one of claims 1 to 4, wherein at least one of the one or more parameters is a depth of the oral care device within the mouth of the user and wherein determining the mouth opening value is further based on the depth of the oral care device within the mouth of the user.

6. The system of any one of claims 1 to 5, wherein the processor is further configured to account for the size of the oral care device when determining the mouth opening value.

7. The system of any one of claims 1 to 6, wherein the processor is further configured to:
track the one or more parameters from the sensor system over time for the user; and
track a progress of the user's mouth opening based on the changes in the one or more parameters over time.

8. The system of claim 7, wherein the processor is further configured to track the one or more parameters from sensors systems in a plurality of oral care devices used by the user, wherein tracking the progress of the user's mouth opening comprises combining the tracked one or more parameters for the plurality of oral care devices, wherein, during the combination, the one or more parameters are weighted based on the corresponding oral care device.

9. The system of any one of claims 1 to 8, wherein the oral care device is a toothbrush, a jetting device or a toothbrush with a jetting device.

10. The system of any one of claims 1 to 9, wherein the sensor system comprises an acoustic sensor for measuring sounds in the mouth of the user, wherein determining the mouth opening value is based on the sounds in the mouth of the user.

11. The system of any one of claims 1 to 10, wherein the sensor system comprises a distance sensor and/or a proximity sensor placed at a distal portion of the oral care device, wherein the distal portion of the oral care device is the portion of the oral care device inserted into the mouth of the user.

12. A computer program product comprising computer program code which, when executed on a computing device having a processing system, cause the processing system to perform all of the following steps:
obtaining one or more parameters, from a sensor system (206, 504) on an oral care device (202, 502), the parameters being of the oral care device or of the user's mouth during use of the oral care device; and
determining a mouth opening value (106, 108) of the user based on the one or more parameter; and
wherein at least one of the one or more parameters is the orientation (602, 604) of the oral care device in the mouth of a user and wherein the processor is further configured to:
detect changes in orientation of the oral care device using the orientation measurements from the sensor system; and
determine the mouth opening value of the user based on the changes in orientation; and/or
wherein at least one of the one or more parameters is the motion of the oral care device in the mouth of a user and wherein the processor is further configured to determine the mouth opening value of the user based on the motion of the oral care device.

## Patentansprüche

1. Mundpflegesystem, umfassend:
eine Mundpflegevorrichtung (202, 502), die ein Sensorsystem (206, 504) umfasst, das dazu konfiguriert ist, einen oder mehrere Parameter der Mundpflegevorrichtung oder des Munds des Benutzers während Verwendung der Mundpflegevorrichtung zu messen; und
einen Prozessor, der dazu konfiguriert ist, den einen oder die mehreren Parameter vom Sensorsystem zu erhalten und basierend auf dem einen oder den mehreren Parametern einen Mundöffnungswert (106, 108) des Benutzers zu bestimmen; und
wobei mindestens einer des einen oder der mehreren Parameter die Ausrichtung (602, 604) der Mundpflegevorrichtung im Mund eines Benutzers ist und wobei der Prozessor weiter für Folgendes konfiguriert ist:
Erfassen von Ausrichtungsänderungen der Mundpflegevorrichtung unter Verwendung der Ausrichtungsmessungen von dem Sensorsystem; und
Bestimmen des Mundöffnungswerts des Benutzers basierend auf den Ausrichtungsänderungen; und/oder
wobei mindestens einer des einen oder der mehreren Parameter die Bewegung der Mundpflegevorrichtung im Mund eines Benutzers ist und wobei der Prozessor weiter dazu konfiguriert ist, den Mundöffnungswert des Benutzers basierend auf der Bewegung der Mundpflegevorrichtung zu bestimmen.

2. System nach Anspruch 1, wobei der Prozessor weiter dazu konfiguriert ist, die Stelle der Mundpflegevorrichtung im Mund des Benutzers zu bestimmen.

3. System nach einem der Ansprüche 1 bis 2, wobei der Prozessor weiter dazu konfiguriert ist, zu bestimmen, ob die Mundpflegevorrichtung an den hintersten Zähnen des Oberkiefers (506) des Benutzers verwendet wird, und wobei Bestimmen des Mundöffnungswerts auf den Messungen von dem Sensorsystem basiert, die dem entsprechen, dass die Mundpflegevorrichtung an den hintersten Zähnen des Oberkiefers des Benutzers verwendet wird.

4. System nach Anspruch 3, wobei der Prozessor, unter Verwendung des einen oder der mehreren Parameter des Sensorsystems, dazu konfiguriert ist, durch Folgendes zu bestimmen, ob die Mundpflegevorrichtung an den hintersten Zähnen des Oberkiefers des Benutzers verwendet wird:
Bestimmen, dass die Mundpflegevorrichtung am Oberkiefer (102) positioniert ist;
Bestimmen, dass sich die Mundpflegevorrichtung in Richtung der Rückseite des Munds bewegt hat; und
Bestimmen, dass die Mundpflegevorrichtung an der okklusalen Seite der hintersten Zähne des Oberkiefers positioniert ist.

5. System nach einem der Ansprüche 1 bis 4, wobei mindestens einer des einen oder der mehreren Parameter eine Tiefe der Mundpflegevorrichtung im Mund des Benutzers ist und wobei Bestimmen des Mundöffnungswerts weiter auf der Tiefe der Mundpflegevorrichtung im Mund des Benutzers basiert.

6. System nach einem der Ansprüche 1 bis 5, wobei der Prozessor weiter dazu konfiguriert ist, beim Bestimmen des Mundöffnungswerts die Größe der Mundpflegevorrichtung zu berücksichtigen.

7. System nach einem der Ansprüche 1 bis 6, wobei der Prozessor weiter für Folgendes konfiguriert ist:
Verfolgen des einen oder der mehreren Parameter des Sensorsystems im Laufe der Zeit für den Benutzer; und
Verfolgen eines Fortschritts der Mundöffnung des Benutzers basierend auf den Änderungen des einen oder der mehreren Parameter im Laufe der Zeit.

8. System nach Anspruch 7, wobei der Prozessor weiter dazu konfiguriert ist, den einen oder die mehreren Parameter von Sensorsystemen in einer Vielzahl von Mundpflegevorrichtungen zu verfolgen, die vom Benutzer verwendet werden, wobei Verfolgen des Fortschritts der Mundöffnung des Benutzers Kombinieren des verfolgten einen oder der mehreren Parameter für die Vielzahl von Mundpflegevorrichtungen umfasst, wobei während der Kombination der eine oder die mehreren Parameter basierend auf der entsprechenden Mundpflegevorrichtung gewichtet werden.

9. System nach einem der Ansprüche 1 bis 8, wobei die Mundpflegevorrichtung eine Zahnbürste, eine Strahlvorrichtung oder eine Zahnbürste mit einer Strahlvorrichtung ist.

10. System nach einem der Ansprüche 1 bis 9, wobei das Sensorsystem einen akustischen Sensor zum Messen von Geräuschen im Mund des Benutzers umfasst, wobei Bestimmen des Mundöffnungswerts auf den Geräuschen im Mund des Benutzers basiert.

11. System nach einem der Ansprüche 1 bis 10, wobei das Sensorsystem einen Abstandssensor und/oder einen Näherungssensor umfasst, der an einem distalen Abschnitt der Mundpflegevorrichtung angebracht ist, wobei der distale Abschnitt der Mundpflegevorrichtung der Abschnitt der Mundpflegevorrichtung ist, der in den Mund des Benutzers eingeführt wird.

12. Computerprogrammprodukt, das Computerprogrammcode umfasst, der, wenn er auf einer Rechenvorrichtung ausgeführt wird, die ein Verarbeitungssystem aufweist, das Verarbeitungssystem dazu veranlasst, alle der folgenden Schritte durchzuführen:
Erhalten eines oder mehrerer Parameter von einem Sensorsystem (206, 504) an einer Mundpflegevorrichtung (202, 502), wobei die Parameter von der Mundpflegevorrichtung oder dem Mund des Benutzers während Verwendung der Mundpflegevorrichtung sind; und
Bestimmen eines Mundöffnungswerts (106, 108) des Benutzers basierend auf dem einen oder den mehreren Parametern; und
wobei mindestens einer des einen oder der mehreren Parameter die Ausrichtung (602, 604) der Mundpflegevorrichtung im Mund eines Benutzers ist und wobei der Prozessor weiter für Folgendes konfiguriert ist:
Erfassen von Ausrichtungsänderungen der Mundpflegevorrichtung unter Verwendung der Ausrichtungsmessungen von dem Sensorsystem; und
Bestimmen des Mundöffnungswerts des Benutzers basierend auf den Ausrichtungsänderungen; und/oder
wobei mindestens einer des einen oder der mehreren Parameter die Bewegung der Mundpflegevorrichtung im Mund eines Benutzers ist und wobei der Prozessor weiter dazu konfiguriert ist, den Mundöffnungswert des Benutzers basierend auf der Bewegung der Mundpflegevorrichtung zu bestimmen.

## Revendications

1. Système de soins buccaux comprenant :
un dispositif de soins buccaux (202, 502) comprenant un système de capteur (206, 504) configuré pour mesurer un ou plusieurs paramètres du dispositif de soins buccaux ou de la bouche de l'utilisateur pendant l'utilisation du dispositif de soins buccaux ; et
un processeur configuré pour obtenir les un ou plusieurs paramètres du système de capteur et déterminer une valeur d'ouverture de bouche (106, 108) de l'utilisateur sur la base des un ou plusieurs paramètres ; et
dans lequel au moins l'un des un ou plusieurs paramètres est l'orientation (602, 604) du dispositif de soins buccaux dans la bouche d'un utilisateur et dans lequel le processeur est en outre configuré pour :
détecter les changements d'orientation du dispositif de soins buccaux à l'aide des mesures d'orientation du système de capteur ; et
déterminer la valeur d'ouverture de bouche de l'utilisateur sur la base des changements d'orientation ; et/ou
dans lequel au moins l'un des un ou plusieurs paramètres est le mouvement du dispositif de soins buccaux dans la bouche d'un utilisateur et dans lequel le processeur est en outre configuré pour déterminer la valeur d'ouverture de bouche de l'utilisateur sur la base du mouvement du dispositif de soins buccaux.

2. Système selon l'une quelconque des revendications 1, dans lequel le processeur est en outre configuré pour déterminer l'emplacement du dispositif de soins buccaux dans la bouche de l'utilisateur.

3. Système selon l'une quelconque des revendications 1 à 2, dans lequel le processeur est en outre configuré pour déterminer si le dispositif de soins buccaux est utilisé sur les dents les plus en arrière de la mâchoire supérieure (506) de l'utilisateur, et dans lequel la détermination de la valeur d'ouverture de bouche est basée sur les mesures provenant du système de capteur qui correspondent au dispositif de soins buccaux utilisé sur les dents les plus en arrière de la mâchoire supérieure de l'utilisateur.

4. Système selon la revendication 3, dans lequel le processeur, à l'aide des un ou plusieurs paramètres provenant du système de capteur, est configuré pour déterminer si le dispositif de soins buccaux utilisé sur les dents les plus en arrière de la mâchoire supérieure de l'utilisateur par :
la détermination du fait que le dispositif de soins buccaux est positionné sur la mâchoire supérieure (102) ;
la détermination du fait que le dispositif de soins buccaux s'est déplacé vers l'arrière de la bouche ; et
la détermination du fait que le dispositif de soins buccaux est positionné sur le côté occlusal des dents les plus en arrière de la mâchoire supérieure.

5. Système selon l'une quelconque des revendications 1 à 4, dans lequel au moins l'un des un ou plusieurs paramètres est une profondeur du dispositif de soins buccaux dans la bouche de l'utilisateur et dans lequel la détermination de la valeur d'ouverture de bouche est en outre basée sur la profondeur du dispositif de soins buccaux dans la bouche de l'utilisateur.

6. Système selon l'une quelconque des revendications 1 à 5, dans lequel le processeur est en outre configuré pour tenir compte de la taille du dispositif de soins buccaux lors de la détermination de la valeur d'ouverture de bouche.

7. Système selon l'une quelconque des revendications 1 à 6, dans lequel le processeur est en outre configuré pour :
suivre les un ou plusieurs paramètres du système de capteur au fil du temps pour l'utilisateur ; et
suivre la progression de l'ouverture de bouche de l'utilisateur sur la base des changements des un ou plusieurs paramètres au fil du temps.

8. Système selon la revendication 7, dans lequel le processeur est en outre configuré pour suivre les un ou plusieurs paramètres à partir de systèmes de capteur dans une pluralité de dispositifs de soins buccaux utilisés par l'utilisateur, dans lequel le suivi de la progression de l'ouverture de bouche de l'utilisateur comprend la combinaison des un ou plusieurs paramètres suivis pour la pluralité de dispositifs de soins buccaux, dans lequel, pendant la combinaison, les un ou plusieurs paramètres sont pondérés sur la base du dispositif de soins buccaux correspondant.

9. Système selon l'une quelconque des revendications 1 à 8, dans lequel le dispositif de soins buccaux est une brosse à dents, un dispositif à jet ou une brosse à dents avec un dispositif à jet.

10. Système selon l'une quelconque des revendications 1 à 9, dans lequel le système de capteur comprend un capteur acoustique pour mesurer les sons dans la bouche de l'utilisateur, dans lequel la détermination de la valeur d'ouverture de bouche est basée sur les sons dans la bouche de l'utilisateur.

11. Système selon l'une quelconque des revendications 1 à 10, dans lequel le système de capteur comprend un capteur de distance et/ou un capteur de proximité placé au niveau d'une partie distale du dispositif de soins buccaux, dans lequel la partie distale du dispositif de soins buccaux est la partie du dispositif de soins buccaux insérée dans la bouche de l'utilisateur.

12. Produit de programme informatique comprenant un code de programme informatique qui, lorsqu'il est exécuté sur un dispositif informatique présentant un système de traitement, amène le système de traitement à mettre en œuvre toutes les étapes suivantes :
obtenir un ou plusieurs paramètres, à partir d'un système de capteur (206, 504) sur un dispositif de soins buccaux (202, 502), les paramètres étant ceux du dispositif de soins buccaux ou de la bouche de l'utilisateur pendant l'utilisation du dispositif de soins buccaux ; et
déterminer une valeur d'ouverture de bouche (106, 108) de l'utilisateur sur la base des un ou plusieurs paramètres ; et
dans lequel au moins l'un des un ou plusieurs paramètres est l'orientation (602, 604) du dispositif de soins buccaux dans la bouche d'un utilisateur et dans lequel le processeur est en outre configuré pour :
détecter les changements d'orientation du dispositif de soins buccaux à l'aide des mesures d'orientation du système de capteur ; et
déterminer la valeur d'ouverture de bouche de l'utilisateur sur la base des changements d'orientation ; et/ou
dans lequel au moins l'un des un ou plusieurs paramètres est le mouvement du dispositif de soins buccaux dans la bouche d'un utilisateur et dans lequel le processeur est en outre configuré pour déterminer la valeur d'ouverture de bouche de l'utilisateur sur la base du mouvement du dispositif de soins buccaux.
